# EUROPEAN PATENT APPLICATION

(11) **EP 4 400 586 A1**
(43) Date of publication of application: **17.07.2024**
(21) Application number: 22867342.2
(22) Date of filing: 06.09.2022
(51) Int. Cl.: C12N 15/09, C07K 19/00, C12N 15/11, C12N 15/12, C12N 15/55, C12N 15/62

(54) **COMPOSITION FOR MODIFYING NUCLEIC ACID SEQUENCE, AND METHOD FOR MODIFYING TARGET SITE OF NUCLEIC ACID SEQUENCE**

(30) Priority: 07.09.2021 JP 2021145726
(71) Applicant: National University Corporation Chiba University, Chiba-shi, Chiba 263-8522 (JP); Bex Co., Ltd., Tokyo 173-0004 (JP)
(72) Inventor: NAKAMURA Ikuo, Chiba-shi, Chiba 263-8522 (JP); MAKABE So, Tokyo 173-0004 (JP); KOBASHI Yuichi, Tokyo 173-0004 (JP); MORIIZUMI Yasuhiro, Tokyo 173-0004 (JP)
(74) Representative: Heyerhoff Geiger GmbH & Co. KG
(86) International application number: PCT/JP2022/033375
(87) International publication number: WO 2023/038020

(57) **Abstract**

An object is to provide a nucleic acid sequence modifying composition and a method for modifying a target site of a nucleic acid sequence that do not depend on a PAM sequence or a PFS sequence. The object can be achieved by a nucleic acid sequence modifying composition including RNA and a fusion protein, in which the RNA includes a hybridization region that may hybridize to a sequence on the 5' side or the 3' side of a target site of a nucleic acid sequence, and a guide region that guides the fusion protein, the guide region includes a recognition region that forms a complex with the fusion protein, and the fusion protein includes a binding domain that recognizes the recognition region of the RNA and forms a complex with the recognition region (however, it is excluded that the binding domain includes an RNA recognition region of a Cas protein group) and a modification domain that modifies the target site of the nucleic acid sequence.

## Description

### [Technical Field]

The disclosure in the present application relates to a nucleic acid sequence modifying composition and a method for modifying a target site of a nucleic acid sequence.

### [Background Art]

In recent years, genome editing that is a technology for modifying target sites of genome DNA in various species has attracted attention. As genome editing methods, the followings are known, for example, (1) a method of performing recombination in a host plant cell or insect cell at a targeted locus in DNA by using a zinc finger nuclease (ZFN) in which a zinc finger DNA binding domain (zinc finger array) and a nonspecific DNA cleavage domain (Nuclease domain) are linked to each other (Patent Literature 1, see FIG. 1A) and (2) a method of cleaving and modifying target genes at a site within or adjacent to a particular nucleotide sequence by using TALEN in which a transcriptional activator-like (TAL) effector of a DNA binding module of plant pathogen Xanthomonas and DNA endonuclease (Nuclease domain) are linked to each other (Patent Literature 2, see FIG. 1B).

As illustrated in FIG. 1A, however, the method disclosed in Patent Literature 1 described above requires two domains (zinc finger array) that recognize genome DNA and are formed of proteins, respectively. Further, two domains that recognize genome DNA are designed so as to recognize the 3' side from a target site of double-stranded DNA, respectively. Therefore, there is a problem that it is required to design a pair of zinc finger arrays having different sequences when the target site of genome DNA is modified.

Further, as illustrated in FIG. 1B, the method disclosed in Patent Literature 2 described above differs from the method disclosed in Patent Literature 1 in that the direction of the domain that recognizes genome DNA (TAL effector) is on the 5' side from the target site of double-stranded DNA. In the same manner as in the method disclosed in Patent Literature 1, however, there is a problem that it is required to design of a pair of TAL effectors having different sequences when the target site of genome DNA is modified.

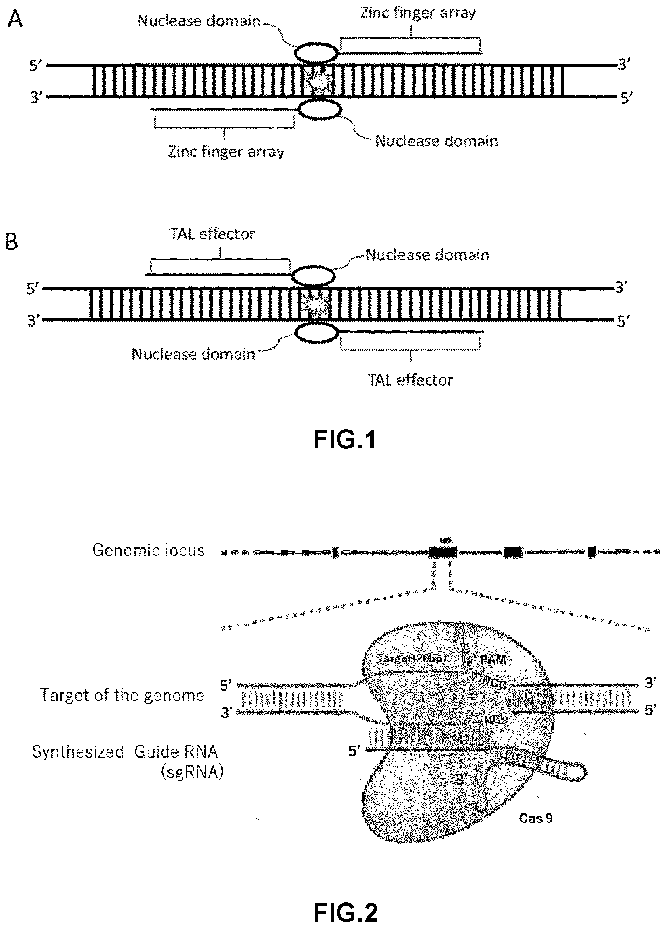

On the other hand, as a technology for modifying a target site of genome DNA, CRISPR/Cas9 has attracted attention in recent years (see Patent Literatures 3 to 6). As illustrated in FIG. 2, CRISPR/Cas9 is formed of guide RNA having a sequence complementary to DNA intended to modify and a Cas9 protein forming a complex with the guide RNA. The guide RNA of CRISPR/Cas9 is only required to recognize only one strand of the double-stranded DNA to form a complementary pair. Therefore, unlike Patent Literatures 1 and 2 described above, there is an advantage that only single guide RNA is sufficient for guiding the nuclease domain that modifies a target site of genome DNA.

### [Citation List]

### [Patent Literature]

Patent Literature 1: Japanese Patent No. 4968498
Patent Literature 2: Japanese Patent Application Laid-Open No. 2013-513389
Patent Literature 3: Japanese Patent Application Laid-Open No. 2015-527889
Patent Literature 4: Japanese Patent Application Laid-Open No. 2016-500262
Patent Literature 5: Japanese Patent Application Laid-Open No. 2016-501531
Patent Literature 6: Japanese Patent Application Laid-Open No. 2016-501532

### [Summary of Invention]

### [Technical Problem]

In the method using CRISPR/Cas9 disclosed in Patent Literatures 3 to 6, however, a base at the third to fourth upstream base of a PAM sequence of genome DNA (5'-NGG-3' or the like) is cleaved by Cas9 endonuclease as illustrated in FIG. 2. That is, the method using CRISPR/Cas9 has a problem of the target site of genome DNA being restricted to a part near the PAM sequence. Further, it is known to modify RNA by using Cas13, however, also in such a case, there is a problem of the target site being restricted to a part near a protospacer flanking site (PFS) sequence.

The disclosure in the present application has been made in order to solve the above problems, and according to an intensive study, it has been newly found that the use of (1) RNA including a hybridization region and a recognition region and (2) a fusion protein forming a complex with the recognition region enables modification of a nucleic acid sequence without depending on a PAM sequence or a PFS sequence. That is, an object of the disclosure in the present application is to provide a nucleic acid sequence modifying composition and a method for modifying a target site of a nucleic acid sequence that do not depend on a PAM sequence or a PFS sequence.

### [Solution to Problem]

(1) A nucleic acid sequence modifying composition comprising RNA and a fusion protein,
   wherein the RNA includes
   a hybridization region adapted to hybridize to a sequence on the 5' side or the 3' side of a target site of a nucleic acid sequence, and
   a guide region that guides the fusion protein,
   wherein the guide region includes at least one recognition region that forms a complex with the fusion protein, and
   wherein the fusion protein includes
      a binding domain that recognizes the recognition region of the RNA and forms a complex with the recognition region, where it is excluded that the binding domain includes an RNA recognition region of a Cas protein group, and
      a modification domain that modifies the target site of the nucleic acid sequence.
(2) The nucleic acid sequence modifying composition according to (1) above, wherein the fusion protein includes a linker sequence that links the binding domain and the modification domain to each other.
(3) The nucleic acid sequence modifying composition according to (1) or (2) above, wherein the guide region includes two recognition regions.
(4) The nucleic acid sequence modifying composition according to any one of (1) to (3) above further comprising a first complementary region connected to one end of the hybridization region,
   wherein the hybridization region and the guide region are indirectly connected to each other when the first complementary region forms a complementary pair with one end side of the guide region.
(5) The nucleic acid sequence modifying composition according to any one of (1) to (4) above, wherein the guide region includes a stem loop.
(6) The nucleic acid sequence modifying composition according to any one of (1) to (5) above,
   wherein the binding domain includes an RNA binding region of Nova, and
   wherein the modification domain includes a cleavage region of FokI that cleaves genome DNA.
(7) The nucleic acid sequence modifying composition according to any one of (1) to (6) above, wherein the nucleic acid sequence is genome DNA.
(8) A nucleic acid sequence modifying composition comprising:
   a nucleic acid that serves as a template used for transcribing the RNA according to any one of (1) to (7) above; and
   a nucleic acid that serves as a template used for translating the fusion protein according to any one of (1) to (7) above.
(9) RNA for forming the nucleic acid sequence modifying composition according to any one of (1) to (7) above.
(10) A nucleic acid that serves as the template used for transcribing the RNA for forming the nucleic acid sequence modifying composition according to (8) above.
(11) A fusion protein for forming the nucleic acid sequence modifying composition according to any one of (1) to (7) above.
(12) A nucleic acid that serves as the template used for translating the fusion protein for forming the nucleic acid sequence modifying composition according to (8) above.
(13) A method for modifying a target site of a nucleic acid sequence, the method comprising:
   an introduction step of introducing the nucleic acid sequence modifying composition according to any one of (1) to (8) above into a cell; and
   a modification step of the modification domain modifying the target site of the nucleic acid sequence.
(14) The method according to (13) above further comprising, before the introduction step, a hybridization region determination step of determining a hybridization region adapted to hybridize to a sequence on the 5' side or the 3' side of the target site of the nucleic acid sequence.
(15) The method according to (13) or (14) above, wherein one hybridization region is required for modifying one target site.
(16) The method according to (15) above, wherein the guide region includes two recognition regions.
(17) The method according to (16) above further comprising, before the introduction step, a guide region designing step of designing at least the number and an RNA sequence of recognition regions so as to be able to form a complex with a required number of fusion proteins for modification of the nucleic acid sequence.

### [Advantageous Effect]

According to the nucleic acid sequence modifying composition and the method for modifying a target site of a nucleic acid sequence disclosed in the present application, it is possible to modify a nucleic acid sequence without depending on a PAM sequence or a PFS sequence.

### [Brief Description of Drawings]

[FIG. 1] FIG. 1A is a schematic diagram illustrating an overview of a genome editing method using zinc finger nuclease. FIG. 1B is a schematic diagram illustrating an overview of a genome editing method using TALEN.
[FIG. 2] FIG. 2 is a schematic diagram illustrating an overview of a genome editing method using CRISPR/Cas9.
[FIG. 3] FIG. 3A and FIG. 3B are schematic diagrams illustrating an overview of a modifying composition 1.
[FIG. 4] FIG. 4A and FIG. 4B are schematic diagrams illustrating an overview of a modified example of the modifying composition 1.
[FIG. 5] FIG. 5 is a diagram illustrating an overview of Example 2.
[FIG. 6] FIG. 6 is a photograph substitute for a drawing, which is a photograph of electrophoresis of Example 2.
[FIG. 7] FIG. 7 is a photograph substitute for a drawing, which is a photograph of electrophoresis to observe a band indicated by the arrow to Lane 3 of Example 2.

### [Description of Embodiments]

Embodiments of a nucleic acid sequence modifying composition (hereafter, which may be referred to as "modifying composition") and a method for modifying a target site of a nucleic acid sequence (hereafter, which may be referred to as "modification method") will be described below in detail with reference to the drawings. Note that, in the present specification, members having the same type of functions are labeled with the same or similar reference numerals. Further, duplicated description for the members labeled with the same or similar reference numerals may be omitted.

Further, the position, size, range, or the like of respective components illustrated in the drawings may be depicted differently from the actual position, size, range, or the like for easier understanding. Thus, the disclosure in the present application is not necessarily limited to the position, size, range, or the like disclosed in the drawings.

### [Embodiment of Nucleic Acid Sequence Modifying Composition]

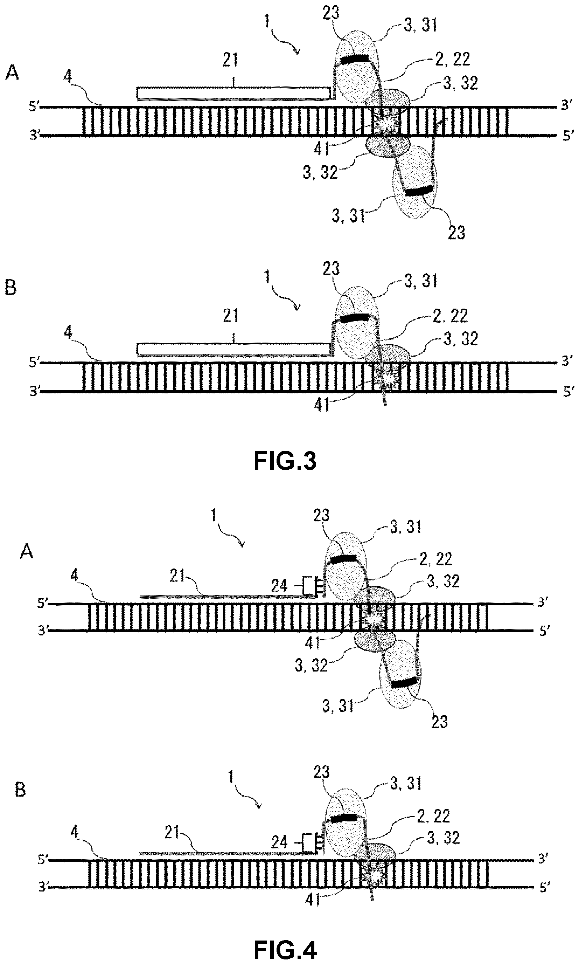

A modifying composition 1 according to an embodiment will be described with reference to FIG. 3 and FIG. 4. FIG. 3A and FIG. 3B are schematic diagrams illustrating the overview of the modifying composition 1. FIG. 4A and FIG. 4B are schematic diagrams illustrating the overview of a modified example of the modifying composition 1. Note that, in the following description, the description common to FIG. 3A and FIG. 3B may be simply denoted as "FIG. 3".

The modifying composition 1 includes RNA 2 and fusion proteins 3. In the example illustrated in FIG. 3, the RNA 2 includes a hybridization region 21 that may hybridize to genome DNA 4 on the 5' side from a target site (modification site) 41 of the genome DNA 4 having double-helix structure and a guide region 22 that guides the fusion proteins 3. Note that, in the example illustrated in FIG. 3, the hybridization region 21 hybridizes to the genome DNA 4 on the 5' side from the target site 41. Alternatively, although not illustrated, the hybridization region 21 may be formed to hybridize to the genome DNA 4 on the 3' side from the target site 41. The guide region 22 is arranged adjacent to the hybridization region 21. Further, the guide region 22 includes recognition regions 23 that recognize binding domains 31 of the fusion proteins 3 to form a complex with the fusion proteins 3. Note that FIG. 3 and FIG. 4 illustrate an example in which the target to be modified by the modifying composition 1 is the genome DNA 4. Alternatively, the modifying composition 1 may modify RNA, which is a nucleic acid sequence. In the following description, although an example in which the target to be modified by the modifying composition 1 is the genome DNA 4 will be described, the same applies to a part other than the description for the modification domain 32 regardless of whether the target to be modified by the modifying composition 1 is the genome DNA 4 or the RNA 4. For the part other than the description for the modification domain 32, the word "genome DNA 4" can be replaced with the word "RNA 4".

Each fusion protein 3 includes the binding domain 31 forming a complex with the recognition region 23 of the RNA 2 and the modification domain 32 that modifies the target site 41 of the genome DNA 4.

The length of the hybridization region 21 is not particularly limited as long as it is a length where the RNA 2 can be positioned on the 5' side or the 3' side of the target site 41 of the genome DNA 4. A shorter length of the hybridization region 21 has a risk of off-target. Therefore, it is preferable to set the length as appropriate taking a risk of off-target into consideration. For example, the length may be, but is not limited to, 15 bp or longer, 20 bp or longer, 25 bp or longer, 30 bp or longer, 35 bp or longer, 40 bp or longer, or 45 bp or longer. On the other hand, in terms of positioning of the RNA 2, although the length of the hybridization region 21 is not particularly limited, a longer hybridization region 21 will cause higher manufacturing cost of the RNA 2. Therefore, the length may be, for example, but is not limited to, 6 kbp or shorter, 5 kbp or shorter, 4 kbp or shorter, 3 kbp or shorter, 2 kbp or shorter, 1 kbp or shorter, 750 bp or shorter, 500 bp or shorter, 300 bp or shorter, 200 bp or shorter, 100 bp or shorter, 90 bp or shorter, 80 bp or shorter, 70 bp or shorter, 60 bp or shorter, or 50 bp or shorter.

It is preferable to adjust the length of the guide region 22 as appropriate taking the size of the fusion protein 3, the distance from the end of the hybridization region 21 to the target site 41, or the like into consideration. Further, the guide region 22 may include a stem loop. In the example illustrated in FIG. 3A, since the target site 41 is modified with the pair of fusion proteins 3 (more specifically, a pair of modification domains 32), the guide region 22 includes two recognition regions 23. When the guide region 22 includes a stem loop, the two recognition regions 23 can be arranged close to each other in a three-dimensional structure. In the example illustrated in FIG. 3A, for example, two stem loops can be formed so that the recognition regions 23 are arranged in the loop portion. Note that the number of stem loops and the portion where the recognition regions 23 are arranged are a mere example. As long as the pair of modification domains 32 are arranged in a positional relationship to enable modification of the target site 41, the number of stem loops may be one, three, or four. Further, the recognition region 23 may be arranged in a stem or may be arranged so as to span the stem and the loop. It is also preferable to adjust the length between the recognition regions 23 (the length of a part of the guide region 22) as appropriate taking the size of the fusion protein 3 or the like into consideration.

The RNA sequence of the recognition region 23 may be determined based on the type of the fusion protein 3 so that the recognition region 23 can form a complex with the binding domain 31 of the fusion protein 3. The combination of the RNA sequence of the recognition region 23 and the binding domain 31 will be described later.

Note that FIG. 3A illustrates the example in which the pair of the modification domains 32 of fusion proteins 3 are arranged at the target site 41 of the genome DNA 4 to modify the target site 41. Therefore, two recognition regions 23 are included in the guide region 22. Alternatively, as illustrated in FIG. 1A and FIG. 1B, a pair of the modifying compositions 1 may be used to hybridize the pair of the hybridization regions 21 to the genome DNA 4 so that the modification domains 32 face the target site 41. This requires designing of two hybridization regions 21 and leads to increased costs or the like but is technically possible. Further alternatively, when a single modification domain 32 of the fusion protein 3 can modify the target site 41, this may require only a single recognition region 23 included in the guide region 22, as illustrated in FIG. 3B. Even with only one recognition region 23 being included in the guide region 22, the guide region 22 may include a stem loop. Further, the recognition region 23 may be arranged in the loop portion, may be arranged in the stem portion, or may be arranged so as to span the stem and the loop. Note that FIG. 3A and FIG. 3B illustrate examples in which each recognition region 23 is a part of the guide region 22. Alternatively, the length of the recognition region 23 may match the length of the guide region 22. In the present specification, that the guide region 22 includes the recognition region 23 means to encompass both of the case where the recognition region 23 is a part of the guide region 22 and the case where the length of the recognition region 23 matches the length of the guide region 22.

The fusion protein 3 is not particularly limited as long as it includes (1) the binding domain 31 that recognizes a specific sequence (the recognition region 23) included in the guide region 22 of the RNA 2 and forms a complex with the recognition region 23 and (2) the modification domain 32 that modifies the target site 41 of the genome DNA 4. Note that, in the present specification, the "fusion protein" is a protein that is artificially synthesized so as to include the binding domain 31 and the modification domain 32 as described above and does not exist naturally. For example, the fusion protein 3 can be made by fusing a protein that can form a complex with the recognition region 23 and a protein that modifies the genome DNA 4. Note that, when a known protein is used to produce the fusion protein 3, it is not required to include all the amino acid sequences of the known protein. For example, in a case of the protein binding to the recognition region 23 of the RNA 2, only the region binding to the recognition region 23 may be used. Further, in a case of a protein that modifies the genome DNA 4, only the region having a function of modifying the genome DNA 4 may be used.

The fusion protein 3 can be produced by a known method. For example, a nucleic acid sequence can be designed based on the amino acid sequence of the designed fusion protein 3, and a cell-free protein synthesis system can be used to synthesize the designed fusion protein 3. Alternatively, a nucleic acid sequence may be designed to include a promoter or the like, the designed nucleic acid sequence may be introduced into plasmid, and the fusion protein 3 may be synthesized by using cells. As described above, in the present specification, when a "fusion protein" is stated, the natural protein itself is excluded.

As described above, the fusion protein 3 is not particularly limited as long as it includes the binding domain 31 and the modification domain 32, and any sequence may be added if necessary. For example, a linker sequence for linking the binding domain 31 and the modification domain 32 to each other or a nuclear localization signal/sequence (NLS) serving as a mark for transporting the fusion protein 3 to cell nuclei may be added. A known sequence may be used for the NLS. When the NLS is added, a position of NLS arrangement is not particularly limited as long as the function achieved by the NLS is obtained. For example, when the fusion protein 3 is formed in the order of the binding domain 31 to the modification domain 32 from upstream when viewed as the amino acid sequence, the NLS may be arranged upstream of the binding domain 31, arranged between the binding domain 31 and the modification domain 32, or arranged downstream of the modification domain 32. Further, when the fusion protein 3 is formed in the order of the modification domain 32 to the binding domain 31 from upstream, the NLS may be arranged upstream of the modification domain 32, arranged between the modification domain 32 and the binding domain 31, or arranged downstream of the binding domain 31.

Further, in the present specification, "modifying" the target site 41 of a nucleic acid sequence (more specifically, the genome DNA 4 or the RNA 4) means turning the state of the target site 41 of double-stranded DNA 4 or single-stranded (double-stranded) RNA 4 into a physically and/or functionally different state from the original state. In other words, it means that the modification domain 32 has a function of turning a nucleic acid sequence into the "different state".

The modification domain 32 that turns the state of the target site 41 of the genome DNA 4 into a physically different state (when being physically different, the function is also often different as a result) may be, for example, but is not limited to, those having the following functions.
(1) Nuclease that hydrolyzes a phosphodiester bond between a sugar and a phosphoric acid of double-stranded DNA into nucleotide
(2) Nickase that introduces nick into one strand of double-stranded DNA
(3) DNA demethylase that demethylates a methyl group of DNA (for example, 5-methylcytosine)
(4) Deaminase that changes NH₂ included in bases of DNA to c=O
(5) Enzyme that substitutes a group of DNA other than the above (3) and (4)

The modification domain 32 that turns the state of the target site 41 of the genome DNA 4 into a functionally different state may be, for example, but is not limited to, those having the following functions.
(6) Transcriptional activator
(7) Transcriptional repressor

More specific examples of the modification domain 32 listed in the above (1) to (7) are illustrated below. Note that enzymes disclosed in Patent Literatures and Non-Patent Literatures may be used as the modification domain 32 without change. Alternatively, as long as the disclosed enzyme achieves a function of modifying the genome DNA 4, some region may be deleted or may be added.

### (1) Nuclease

(1-1) FokI (Japanese Patent No. 5266210; T. Sakuma et al., "Repeating pattern of non-RVD variations in DNA-binding modules enhances TALEN activity", Scientific Reports 3, Article number:3379 (2013))
(1-2) FirmCut nuclease (International Publication No. 2020/045281)

### (2) Nickase

(2-1) RuvC nuclease domain D10A point mutation (M. Jinek et al., "A Programmable Dual-RNA-Guided DNA Endonuclease in Adaptive Bacterial Immunity", Science (2012), Vol. 337, Issue 6096, pp. 816-821; it is also available from WAKENYAKU CO., LTD.)

This enzyme is produced such that the 10th amino acid of Cas9 is changed from aspartic acid (D) to alanine (A). Since nuclease activity of the RuvC-like domain of the two nuclease domains of Cas9 is lost, the D10A mutant acts as nickase and generates single-stranded DNA cleavage (nick) rather than double-stranded DNA cleavage. In a double nicking method, two sgRNA and Cas9 nickase to be guided to each strand of the target sequences of double-stranded DNA are caused to act. (2-2) HNH nuclease domain H840A point mutation (see the references disclosed in the above (2-1); it is also available from WAKENYAKU CO., LTD.)

While the enzyme listed in the above (2-1) cleaves only the target strand of DNA, the enzyme listed in the above (2-2) cleaves the non-target strand.

### (3) DNA demethylase

(3-1) Methylated cytosine hydroxylase Tet1 (S. Morita et al., "Targeted DNA demethylation in vivo using dCas9-peptide repeat and scFv-TET1 catalytic domain fusions", Nature Biotechnology 34, p:1060-1065 (2016))

### (4) Deaminase

(4-1) Adenosine deaminase (C. Li et al., "Expanded base editing in rice and wheat using a Cas9-adenosine deaminase fusion", Genome Biology 19, Article number:59 (2018))
(4-2) Cytidine deaminase (A. Komor et al., "Programmable editing of a target base in genomic DNA without double-stranded DNA cleavage", nature 533, p:420-424 (2016))

### (5) Other enzymes

Histone acetyltransferase, histone deacetylase, histone lysine methyltransferase, histone lysine demethyltransferase, and the like

### (6) Transcriptional activator

(6-1) Vp64 (L. Lowder et al., "Robust Transcriptional Activation in Plants Using Multiplexed CRISPR-Act 2.0 and mTALE-Act Systems", Molecular Plant, Volume 11, Issue 2, 2018, p:245-256)
(6-2) Vp16 (see the reference listed in the above (6-1))

### (7) Transcriptional repressor

(7-1) KRAB (M. Boettcher et al., "Choosing the Right Tool for the Job: RNAi, TALEN, or CRISPR", Molecular Cell, 2015 21; 58(4) :575-85)
(7-2) SRDX(L. Lowder et al., "A CRISPR/Cas9 Toolbox for Multiplexed Plant Genome Editing and Transcriptional Regulation", Plant Physiology, 2015, 169(2) :971-85)

Further, although partially duplicated with the above (1) to (7), "transposase domain, integrase domain, recombinase domain, resolvase domain, invertase domain, protease domain, DNA methyltransferase domain, DNA hydroxymethylase domain, DNA demethylase domain, histone acetylase domain, histone deacetylase domain, nuclease domain, repressor domain, activator domain, nuclear localization signal domain, transcription-regulatory protein (or transcription complex recruitment) domain, cellular uptake activity-related domain, nucleic acid binding domain, antibody presentation domain, histone modifying enzymes, recruiter of histone modifying enzymes; inhibitors of histone modifying enzymes, histone methyltransferases, histone demethylases, histone kinases, histone phosphatases, histone ribosylase, histone deribosylase, histone ubiquitinase, histone deubiquitinase, histone biotinase, and histone tail protease" or the like disclosed in Japanese Patent Application Laid-Open No. 2015-527889 may be used. The disclosed features of Japanese Patent Application Laid-Open No. 2015-527889 are incorporated in the present specification by reference.

Among the enzymes that can form the modification domain 32 illustrated above as examples, the enzyme that modifies the genome DNA 4 by two modification domains 32 as illustrated in FIG. 3A, the enzyme that modifies the genome DNA 4 by one modification domain 32 as illustrated in FIG. 3B, or the enzyme that modifies the genome DNA 4 by four modification domains may be of the following types, for example.

### <One modification domain 32>

RuvC nuclease domain D10A point mutation, HNH nuclease domain H840A point mutation

### <Two modification domains 32>

FokI, adenosine deaminase, cytidine deaminase, Vp16

### <Four modification domains 32>

### VP64

Further, the modification domain 32 that turns the state of the target site 41 of the RNA 4 into a functionally different state may be, for example, but is not limited to, Adenosine Deaminase Acting on RNA (ADAR) (Marina et al., "Evaluation of Engineered CRISPR-Cas-Mediated Systems for Site-Specific RNA Editing", Cell Reports 33, 108350, November 3, 2020), which is double-stranded RNA specific adenosine deaminase, or the like.

The sequence of the binding domain 31 and the recognition region 23 is not particularly limited as long as it is a combination of a protein that can recognize the recognition region 23 of the RNA 2 and form a complex with the recognition region 23 and an RNA sequence. Therefore, when the guide region 22 includes two or more recognition regions 23, the recognition region 23 having the same RNA sequence 31 may be included for the same binding domain 31, or the recognition region 23 having different RNA sequences may be included for the same binding domain 31. For the same reason, when the guide region 22 includes two or more recognition regions 23, the same binding domain 31 may form a complex for the same RNA sequence (recognition region 23), or different binding domains 31 may form a complex for the same RNA sequence (recognition region 23).

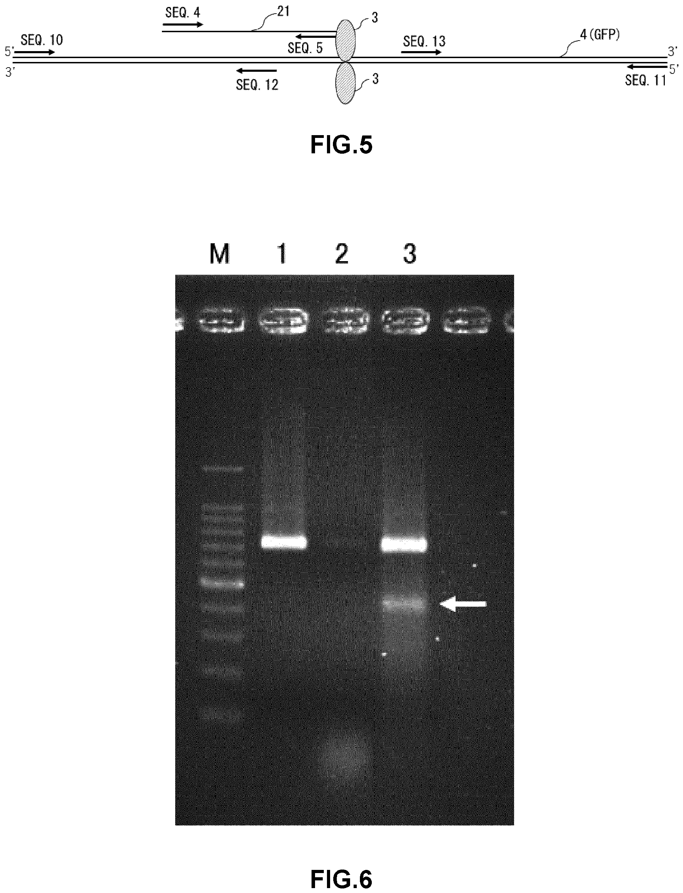

The sequence of the binding domain 31 and the recognition region 23 may be, for example, but is not limited to, the following combinations.
(1) Nova: 5'-UCAY-3' (K. Jensen et al., "The tetranucleotide UCAY directs the specific recognition of RNA by the Nova K-homology 3 domain", PNAS, 2000, 97 (11), 5740-5745, and hereafter, this paper may be referred to as "Non-Patent Literature 1")
Note that the above 5'-UCAY-3' is formed in the loop portion of the stem loop.
(2) Combinations disclosed in FIG. 6 of Non-Patent Literature 1 stated above (see Table 1 below)

**[Table 1]**

| No. | RNA binding protein name | RNA name | RNA sequence |
|---|---|---|---|
| 1 | Nova-1 | α2-glyR | See sequence element in FIG.6 |
| 2 | Nova (KH3) | 10021 | See sequence element in FIG.6 |
| 3 | ZBP-1 | β-actin | See sequence element in FIG.6 |
| 4 | hnRNP-E/hnRNP-K | 15-LOX | See sequence element in FIG.6 |
| 5 | hnRNP-E | α-globin | See sequence element in FIG.6 |
| 6 | BBP/SF1 | branchpt. | See sequence element in FIG.6 |

(3) TLS:5'-GGUG-3'(Wang et al.,"Induced ncRNAs allosterically modify RNA-binding proteins in cis to inhibit transcription",nature,Vol 454,3,July 2008)
Further, although the RNA sequence is not explicitly indicated, proteins binding to RNA may be the following proteins. Since it is well known to bind to RNA, the RNA sequence can be designed as appropriate.
(4) RNG105(N. Shiinaet et al.,"A Novel RNA-Binding Protein in Neuronal RNA Granules: Regulatory Machinery for Local Translation",The Journal of Neuroscience,April 27,2005·25(17):4420-4434)
(5) NAPOR(W. ZHANGet et al.,"Region-specific alternative splicing in the nervous system: Implications for regulation by the RNA-binding protein NAPOR",RNA(2002),8:671-685) .Cambridge University Press)
(6) DAZL and RBFOX(D. Sharma1 et al., "The kinetic landscape of an RNA-binding protein in cells" ,Nature,Vol 591,4 March 2021)
(7) Sam68(P. Bielli et al.,"The RNA-binding protein Sam68 is a multifunctional player in human cancer",Endocrine-Related Cancer (2011) 18 R91-R102)
(8) Spi-1/PU.1(M. Hallier et al.,"The Transcription Factor Spi-1/PU.1 Binds RNA and Interferes with the RNA-binding Protein p54nrb"),THE JOURNAL OF BIOLOGICAL CHEMISTRY,Vol.271,No.19,pp.11177-11181,1996)
(9) PUM2 Protein, QKI Protein,IGF2BP Protein(M. Hafner et al., "Transcriptome-wide Identification of RNA-Binding Protein and MicroRNA Target Sites by PAR-CLIP" ,Cell,141,129-141,2010)

The disclosure of each paper cited for indicating specific examples of the binding domain 31 and the modification domain 32 described above and the disclosure of paper cited by each paper are incorporated in the present specification by reference.

Note that it is well known that Cas9 and the like, which are the Cas protein group, recognize a PAM sequence of the genome DNA 4. Further, it is also well known that PAM sequences differ in accordance with bacterial species from which nuclease is derived and the type/subtype of nuclease (see Table 2 below). Furthermore, it is well known that Cas13, which is the Cas protein group, recognizes the PFS sequence of RNA. It is therefore excluded that the binding domain 31 disclosed in the present application includes a protein that recognizes and binds to a PAM sequence or a PFS sequence, and a region that recognizes a PAM sequence or a PFS sequence of the protein. Alternatively, it may be rephrased in that it is excluded that the binding domain 31 includes an RNA recognition region of a Cas protein group. The Cas protein group may be, for example, Cas9, Cpf1, Cas3, Cas8a-c, Cas10, Cse1, Csy1, Csn2, Cas4, Csm2, Cm5, or the like. On the other hand, among the Cas protein group, the region that modifies a nucleic acid sequence may be included as the modification domain 32.

**[Table 2]**

| **strain** | **subtype** | **PAM sequence** |
|---|---|---|
| *S. pyogenes* | II | 5'-NGG |
| *S. aureus* | II-A | 5'-NNGRRT |
| *S. solfataricus* | I-A1 | 5'-CCN |
| *S. solfataricus* | I-A2 | 5'-TCN |
| *H. walsbyi* | I-B | 5'-TTC |
| *E. coli* | I-E | 5'-AWG |
| *E. coli* | I-F | 5'-CC |
| *P. aeruginosa* | I-F | 5'-CC |
| *S. thermophilus* | II-A | 5'-NNAGAA |
| *S. agalactiae* | II-A | 5'-NGG |
| *F. novicida* | V-A | TTTN-'3 |
| *Acidaminococcus sp.* | V-A | TTTN-'3 |

The use of the modifying composition according to the first embodiment achieves the following advantageous effects.
(1) The RNA 2 includes the hybridization region 21 that hybridizes to the genome DNA 4 or RNA 4 and the guide region 22. Further, the guide region 22 includes one or more recognition regions 23 that form a complex with the fusion protein 3. Therefore, as illustrated in FIG. 3A and FIG. 3B, only one hybridization region 21 is required regardless of whether it is a monomer or a dimer (or a higher multimer) by which the modification domain 32 has the modification function. This facilitates the design of the hybridization region 21.
(2) In the CRISPR/Cas9 disclosed in Patent Literatures 3 to 6, the target site 41 of the genome DNA 4 is restricted to a part near the PAM sequence. Further, also in a case of Cas13, the target site 41 of the RNA 4 is restricted to a part near the PFS sequence. In contrast, in the modifying composition 1 according to the first embodiment, the target site 41 can be determined without restriction of the sequence of the genome DNA 4 or the RNA 4.
(3) The number of recognition regions 23 included in the guide region 22 can be designed to be any number. Therefore, the guide region 22 can be designed in accordance with the type of the fusion protein 3, and this improves flexibility in the design of the guide region 22.
(4) Unlike natural proteins (and partially modified proteins), the fusion protein 3 can be formed by selecting and fusing a region functioning as the binding domain 31 and a region functioning as the modification domain 32 each other. Therefore, the size of the fusion protein 3 can be reduced compared with the conventional natural proteins (and partially modified proteins). Furthermore, flexibility in the design of the fusion protein 3 is improved.

### <Modified Example 1 of Nucleic Acid Sequence Modifying Composition>

Next, Modified example 1 of the modifying composition 1 according to the embodiment will be described with reference to FIG. 4A and FIG. 4B. In the example illustrated in FIG. 3, the hybridization region 21 and the guide region 22 of the RNA 2 are in direct contact with each other. In contrast, in the modified examples illustrated in FIG. 4A and FIG. 4B, the RNA 2 includes a first complementary region 24 connected to one end of the hybridization region 21. Further, the first complementary region 24 forms a complementary pair with the one end side of the guide region 22, and thereby the hybridization region 21 and the guide region 22 are connected indirectly to each other.

In the case of the modifying composition 1 according to the embodiment, since the hybridization region 21 and the guide region 22 are in direct connection with each other, it is required to form the RNA 2 in an integrated manner. In contrast, in Modified example 1, only the hybridization region 21 and the first complementary region 24 are required to be formed in accordance with the target site 41. Therefore, costs can be reduced by mass production of the guide regions 22 and the fusion proteins 3. Further, a business entity that provides the modifying composition 1 may provide only the guide regions 22 and the fusion proteins 3, and it is possible to design and synthesize the hybridization region 21 and the first complementary region 24 on the user side, which allows the user to start an experiment promptly.

### <Modified Example 2 of Nucleic Acid Sequence Modifying Composition>

Next, Modified example 2 of the modifying composition 1 according to the embodiment will be described. In the modifying composition 1 according to the above embodiment and Modified example 1, the modifying composition 1 is formed of the RNA 2 and the fusion protein 3. Alternatively, as Modified example 2, the modifying composition 1 may be formed by using a nucleic acid serving as a template for transcribing the RNA 2 (hereafter, which may be referred to as "template for the RNA 2") and a nucleic acid serving as a template for translating the fusion protein 3 (hereafter, which may be referred to as "template for the fusion protein 3"). DNA or RNA can be used for the template for the RNA 2 and the template for the fusion protein 3. The template for the RNA 2 is required to include at least a sequence for transcribing the RNA 2. Further, the template for the fusion protein 3 is required to include at least a sequence for translating the fusion protein 3. The template for the RNA 2 and the template for the fusion protein 3 may include a promoter or an untranslated region used for promoting translation if necessary.

More specifically, when the template for the RNA 2 and the template for the fusion protein 3 are DNA, a promoter for RNA transcription is linked upstream of the template for the RNA 2, and a promoter for protein translation is linked upstream of the template for the fusion protein 3. Further, once the template for the RNA 2 and the template for the fusion protein 3 are introduced into a cell, then, in the cell, (1) the RNA 2 is transcribed from the template for the RNA 2, (2) mRNA is transcribed from the template for the fusion protein 3, and the fusion protein 3 is translated from the transcribed mRNA, and (3) the modifying composition 1 is formed in the cell.

Further, when the template for the RNA 2 and the template for the fusion protein 3 are DNA, these templates may be inserted in the same plasmid vector together with a promoter or may be inserted in different plasmid vectors. Alternatively, the template for the RNA 2 and the template for the fusion protein 3 may be inserted in a DNA type virus such as an adeno-associated virus (AAV) together with a promoter instead of a plasmid vector. Once the plasmid vector or the DNA type virus is introduced into a cell, the modifying composition 1 is formed in the cell. Note that, when the plasmid vector or the DNA type virus has already included a promoter, the template for the RNA 2 and the template for the fusion protein 3 that include no promoter can be inserted in the plasmid vector or the DNA type virus.

When the template for the RNA 2 and the template for the fusion protein 3 are RNA, an RNA type virus such as a lentivirus can be used instead of the DNA type virus. When the RNA type virus is used, DNA is first reverse-transcribed from the template for the RNA 2 and the template for the fusion protein 3 inserted in the RNA type virus introduced into a cell. The RNA 2 is then transcribed from the reverse-transcribed DNA, and mRNA is transcribed from the reverse-transcribed DNA, and the fusion protein 3 is translated from the mRNA. Note that the template for the fusion protein 3 may be mRNA. When the template for the fusion protein 3 is mRNA, it is not required to link a promoter to mRNA. Once mRNA is introduced into a cell, the fusion protein 3 can be directly translated from mRNA in the cell.

As described above, the path from which the RNA 2 and the fusion protein 3 are finally obtained differs in accordance with the type of nucleic acid serving as a template. In the present specification, when "nucleic acid serving as a template for transcribing RNA" is stated, this represents a concept that the nucleic acid serving as a template includes a template that indirectly transcribes the RNA 2 (template RNA) in addition to the template that directly transcribes the RNA 2 (template DNA). Similarly, when a "nucleic acid serving as a template for translating a fusion protein" is stated, this represents a concept that the nucleic acid serving as a template includes a template that indirectly translates the fusion protein 3 (template DNA, template RNA) in addition to the template that directly translates the fusion protein 3 (mRNA).

Note that, for the template for the RNA 2 and the template for the fusion protein 3, the type (template DNA, template RNA, mRNA) and the form (whether or not there is insertion into a vector or a virus) may be the same or may be different as long as the modifying composition 1 is finally formed in a cell.

The promoter for RNA transcription, the promoter for protein translation, the plasmid, the DNA type virus, and the RNA type virus are not particularly limited as long as the functions described above are achieved, and those that are known can be used. For example, the promoter for RNA transcription may be U6 Promoter or the like, but is not limited thereto. The promoter for protein translation may be CMV Promoter or the like. The plasmid may be pcDNA3.1 for mammals, pBluescriptII KS and pET system for bacteria; pPIC system for yeasts, or the like. The DNA type virus may be the adeno-associated virus (AAV) described above. The RNA type virus may be the lentivirus or the like described above. Note that the modifying composition 1 according to Modified example 2 may be rephrased as a composition for forming a modifying composition.

### <Modified Example 3 of Nucleic Acid Sequence Modifying Composition>

Next, Modified example 3 of the modifying composition 1 according to the embodiment will be described. In the modifying composition 1 according to the embodiment described above and Modified example 1, the RNA 2 and the fusion protein 3 form the modifying composition 1 in combination. Further, in Modified example 2, the template for the RNA 2 and the template for the fusion protein 3 form the modifying composition 1 in combination. Alternatively, in Modified example 3, only one of the combined elements forming the modifying composition 1 is provided, and thereby the modifying composition 1 may be formed by combining the separately provided elements in use.

More specifically, (1) only the RNA 2 described in the modifying composition 1 according to the embodiment or described in the Modified example 1 is provided as the RNA 2 used for forming the modifying composition 1, (2) only the fusion protein 3 described in the modifying composition 1 according to the embodiment or described in the Modified example 1 is provided as the fusion protein 3 used for forming the modifying composition 1, (3) only the template for the RNA 2 described in Modified example 2 is provided as the template for the RNA 2 used for forming the modifying composition 1, and (4) only the template for the fusion protein 3 described in Modified example 2 is provided as the template for the fusion protein 3 used for forming the modifying composition 1. The RNA 2 and the fusion protein 3 have already been described in the modifying composition 1 according to the embodiment and Modified example 1, and the template for the RNA 2 and the template for the fusion protein 3 have already been described in Modified example 2. Thus, the specific description thereof will be omitted to avoid duplicated description.

### <Embodiment of Method for Modifying Target Site of Nucleic Acid Sequence>

The modification method includes an introduction step of introducing, into a cell, the modifying composition 1 described in the embodiment and the modified examples of the above modifying composition and a modification step of the modification domain 32 modifying the target site 41 of the nucleic acid sequence 4.

The cell is not particularly limited as long as it includes the nucleic acid sequence 4. The cell may be, for example, human or non-human animal cells; plant cells; insect cells; microbial cells such as E. coli, yeasts, molds; or the like. Further, the cell may be a single cell or a mass of aggregated cells (spheroid). In the present specification, when a "cell" is stated, this encompasses both concepts of a single cell and a mass of multiple aggregated cells.

The introduction step is not particularly limited as long as it is possible to introduce the modifying composition 1 into a cell, and a known method such as electroporation may be used. Further, when a DNA virus or an RNA virus is used as the modifying composition according to Modified example 2, a cell can be infected via a known method. Note that, when the modifying composition 1 according to Modified example 2 is used, a step of the RNA 2 and the fusion protein 3 being formed in the cell is included after the introduction step.

In the modification step, the hybridization region 21 of the modifying composition 1 introduced into a cell hybridizes to the nucleic acid sequence 4, and the modification domain 32 modifies the target site 41 of the nucleic acid sequence 4. Note that, as described above, the number of hybridization regions 21 required for modifying one target site 41 may be one or two. However, the modifying composition 1 disclosed in the present application can include two or more recognition regions 23 in one guide region 22. Therefore, even with one hybridization region 21 required for modifying one target site 41, it is possible to design the recognition region 23 so as to form a complex with a required number of fusion proteins 3 for modification of the nucleic acid sequence 4. In a case of one hybridization region 21 required for modifying one target site 41, the manufacturing cost can be reduced, and convenience of experiment can be improved.

The modification method may include, before the introduction step, a hybridization region determination step of determining the hybridization region 21 that may hybridize to the sequence on the 5' side or the 3' side of the target site 41 of the nucleic acid sequence 4. The entire RNA 2 may be produced to include the hybridization region 21 determined in the hybridization region determination step. Further, the modifying composition 1 may be produced and the modification method may be implemented by preparing the hybridization region 21 and the first complementary region 24 on the user side and combining therewith the guide region 22 and the fusion protein 3 that are separately provided.

The modification method may include, before the introduction step, a guide region designing step of designing at least the number and the RNA sequence of recognition regions 23 so that a complex can be formed with the required number of fusion proteins 3 for modification of the nucleic acid sequence 4. The guide region designing step may include designing of a linker sequence that links to two or more recognition regions 23 if necessary. It has not been known that one guide region 22 forms a complex with two or more fusion proteins 3 and the modification domains 32 included in the fusion proteins 3 modify the nucleic acid sequence 4. Therefore, the guide region designing step described above is a novel step.

Further, DNA has a self-repair function. Therefore, when the nucleic acid sequence 4 is genome DNA, a step of introducing DNA into the target site 41 may be performed, if necessary, after the modification step is performed. When the modification domain 32 is an enzyme that cleaves the genome DNA 4, such as FokI, the target site 41 of the genome DNA 4 is cleaved and modified into a physically different state by the modification step. As a result, the genome DNA 4 may lose the function (knock out). On the other hand, the cleaved genome DNA 4 may repair the cleaved portion by the self-repair function. By introducing ssODN, ssDNA, or dsDNA into a cell together with the modifying composition 1 at the same time, it is possible to insert a desired DNA fragment into the cleaved portion when repairing the target site 41 of the genome DNA 4 cleaved by the modification step. By inserting a desired DNA fragment, it is also possible to add an intended function to the genome DNA 4 (knock in).

Note that the disclosure in the present application is not limited to the embodiments described above. Any combination of each embodiment described above or modification of any component or omission of any component in each embodiment is possible within the scope of the disclosure in the present application.

Although Examples will be presented below to specifically describe the embodiment disclosed in the present application, these Examples are only for the purpose of illustration of the embodiment and are not intended to limit or restrict the scope of the invention disclosed in the present application.

### [EXAMPLE]

### <Example 1>

The modifying composition 1 was produced in accordance with the following procedure.

### (1) Production of a fusion protein (FokI-Nova)

As the fusion protein 3, FokI-Nova was produced. The amino acid sequence of FokI-Nova is indicated in SEQ. ID. 1 (sequence No. 1). Note that, in SEQ. ID. 1 indicated in Table 3 below, the underlined part ("Q" at the seventh character from the right on the first line to "F" at the eleventh character from the left on the fifth line) represents the cleavage region of FokI (the modification domain 32), the double-underlined part ("K" at the 28th character from the left on the fifth line to "G" at the eighth character from the right on the sixth line) represents the RNA binding region of NOVA (the binding domain 31), and the bold underlined part ("P" at the third character from the right on the sixth line to "V" at the fourth character from the left on the seventh line) represents the NLS. The remaining is a linker sequence or the like.

**[Table 3]**

| NAME | SEQUENCE | SEQ. ID. |
|---|---|---|
| FokI-Nova | MHHHHHHSSGLVPRGSGMKETAAAKFERQHMDSPDLGTDDD DKAMADIGSQLVKSELEEKKSELRHKLKYVPHEYIELIEIA RNSTQDRILEMKVMEFFMKVYGYRGKHLGGSRKPDGAIYTV GSPIDYGVIVDTKAYSGGYNLPIGQADEMQRYVEENQTRNK HINPNEWWKVYPSSVTEFKFLFVSGHFKGNYKAQLTRLNHI TNCNGAVLSVEELLIGGEMIKAGTLTLEEVRRKFNNGEINF SGSETPGTSESATPESKDVVEIAVPENLVGAILGKGGKTLV EYQELTGARIQISKKGEFVPGTRNRKVTITGTPAATQAAQY LITQRITYEQGVRAANPQKVGSRADPKKKRKV | 1 |

### (1-1) Cloning of an expression vector into E. coli for expression

An expression vector designed so as to express the FokI-Nova indicated in sequence No. 1 was cloned into E. coli for expression. Then, 1 µL of expression vector adjusted to a concentration of 10 ng/uL and 50 µL of E. coli for expression were mixed and allowed to stand on ice for 20 minutes. Then, the mixture was subjected to heat shock in treatment at 42 °C for 1 minute and then returned to be placed on the ice. The whole transformed E. coli and 250 µL of SOC medium were mixed and cultured at 37 °C for 1 hour. From this mixture, 100 µL thereof was plated on an LB plate (containing antibiotics) and cultured at 37 °C overnight.

A single colony was picked up from the plate and cultured at 37 °C overnight. The same amount of the cultured solution and 50% glycerol solution were mixed and then stored at -80 °C.

### <Reagent>

- E. coli for expression: BL21(DE3)pLysS
- Antibiotic: Kanamycin

### (1-2) Culturing and purification with 100 mL

Bacteria were inoculated from E. coli glycerol stock and cultured in 10 ml of LB medium (containing antibiotics) at 37 °C overnight. Then, 10 ml of the previous cultured solution was added to 100 mL of LB medium (containing antibiotics) and cultured at 37 °C. It was confirmed that OD was around 0.6, IPTG was added to have the final concentration of 1 mM, and the mixture was cultured at 25 °C overnight. The bacteria were suspended with 5 ml of protein extraction reagent. The protein extraction reagent, 10 µl of Lysonase Bioprocessing Reagent (Novagen) was added, and the mixture was stirred at room temperature for 5 minutes. The mixture was centrifuged to collect supernatant as a soluble fraction. Then, 1 ml of resin was loaded to a column for precipitation. After the stored liquid was drained, 10 bed volumes of equilibrium buffer was added to equilibrate the resin.

The soluble fraction was loaded to the column to bind a His-Tag fusion protein. The column was washed with 8 bed volumes of equilibrium buffer. The column was washed with 7 bed volumes of equilibrium buffer (containing 10 mM of Imidazole). The His-Tag fusion protein was eluted with 3 bed volumes of elution buffer.

The purified protein was mixed with the same amount of EzApply (ATTO) and subjected to thermal treatment at 95 °C for 5 minutes to prepare an electrophoresis sample. The sample was applied to 5 to 20% polyacrylamide gel and subjected to electrophoresis at 20 mA for 70 minutes. The FokI antibody was used to confirm that a FokI-Nova fusion protein having the designed size was synthesized.

### <Reagent>

- Protein extraction reagent: BugBuster Protein Extraction Reagent (Novagen)
- Purification resin: TALON Metal Affinity Resin (Takara Bio)
- Equilibrium buffer: 50 mM sodium phosphate, 300 mM sodium chloride; pH 7.4
- Elution buffer: 50 mM sodium phosphate, 300 mM sodium chloride, 150 mM imidazole; pH 7.4
- Electrophoresis buffer: 25 mM Tris pH 8.3, 192 mM Glycine, 0.1% SDS

(2) Production of RNA 2 (Hybridization Region 21 + Guide Region 22)
The sequence of the RNA 2 produced in Examples is indicated in SEQ. ID. 2. The RNA 2 was produced by the following procedure.
(a) Hybridization Region 21
   pEGFP-N1 (SEQ. ID. 3) is used as a template,
   - Fw primer (eGFP Xba5P: 5'-GCTCTAGAAAACGGCCACAAGTTCAGCGTGTC-3': SEQ. ID. 4)
   - Rv primer (eGFP Spe3P: 5'-TGACTAGTGGGTGTCGCCCTCGAACTTCACCT-3': SEQ. ID. 5)
   were used for amplification of 290 bp, and restriction enzyme sites were added to both ends. Ligation was performed on the pCR2.1 vector by an ordinary method with XbaI and SpeI.

Note that the underlined parts of SEQ. IDs. 4 and 5 are sequences forming a complementary pair with the DNA sequence 4 coding the GFP illustrated in FIG. 5 (both ends of the hybridization region 21). Note that, in FIG. 5, depiction of "ID." for sequence numbers is omitted due to space limitation.
(b) Guide Region 22
   UCAY5P (5'-TCGGATCCGCAGTCTCATCATCATTTTCATTTTGTTCGTTAGCACATTGGGCAGT CTCAT-3': SEQ. ID. 6) and UCAY3P (5'-GAAGATCTCAAAATGAAAATGATGATGAGACTGCCCAATGTGCTAACGAACAAAA TGAAA-3': SEQ. ID. 7) were annealed and subjected to an elongation reaction. A template annealed on a BamHI site was subjected to restriction enzyme treatment with BamHI and BglII, and ligation was performed on the downstream of the above (a) by an ordinary method.
(c) Synthesis of RNA 2
   Restriction enzyme treatment was performed on the pCR2.1 vector with HindIII, and RNA was synthesized with in vitro Transcription T7 Kit.

Note that the RNA 2 includes two α2-glyR (GCAGUCUCAUCAUCAUUUUCAUUUUG: SEQ. ID. 8) of FIG. 6 of Non-Patent Literature 1 as the recognition regions 23 via a linker.

**[Table 4]**

| NAME | SEQUENCE | SEQ. ID. |
|---|---|---|
| RNA2 | GGGCGAAUUGGGCCCUCUAGAAAACGGCCACAAGUUCAGCG UGUCCGGCGAGGGCGAGGGCGAUGCCACCUACGGCAAGCUG ACCCUGAAGUUCAUCUGCACCACCGGCAAGCUGCCCGUGCC CUGGCCCACCCUCGUGACCACCCUGACCUACGGCGUGCAGU GCUUCAGCCGCUACCCCGACCACAUGAAGCAGCACGACUUC UUCAAGUCCGCCAUGCCCGAAGGCUACGUCCAGGAGCGCAC CAUCUUCUUCAAGGACGACGGCAACUACAAGACCCGCGCCG AGGUGAAGUUCGAGGGCGACACCCACUAGUGGAUCCGCAGU CUCAUCAUCAUUUUCAUUUUGUUCGUUAGCACAUUGGGCAG UCUCAUCAUCAUUUUCAUUUUGAGAUCCGAGCUCGGUACCA AGCU | 2 |
| pEGF P-N1 | TAGTTATTAATAGTAATCAATTACGGGGTCATTAGTTCATA GCCCATATATGGAGTTCCGCGTTACATAACTTACGGTAAAT GGCCCGCCTGGCTGACCGCCCAACGACCCCCGCCCATTGAC GTCAATAATGACGTATGTTCCCATAGTAACGCCAATAGGGA CTTTCCATTGACGTCAATGGGTGGAGTATTTACGGTAAACT GCCCACTTGGCAGTACATCAAGTGTATCATATGCCAAGTAC GCCCCCTATTGACGTCAATGACGGTAAATGGCCCGCCTGGC ATTATGCCCAGTACATGACCTTATGGGACTTTCCTACTTGG CAGTACATCTACGTATTAGTCATCGCTATTACCATGGTGAT GCGGTTTTGGCAGTACATCAATGGGCGTGGATAGCGGTTTG ACTCACGGGGATTTCCAAGTCTCCACCCCATTGACGTCAAT GGGAGTTTGTTTTGGCACCAAAATCAACGGGACTTTCCAAA ATGTCGTAACAACTCCGCCCCATTGACGCAAATGGGCGGTA GGCGTGTACGGTGGGAGGTCTATATAAGCAGAGCTGGTTTA GTGAACCGTCAGATCCGCTAGCGCTACCGGACTCAGATCTC GAGCTCAAGCTTCGAATTCTGCAGTCGACGGTACCGCGGGC CCGGGATCCACCGGTCGCCACCATGGTGAGCAAGGGCGAGG AGCTGTTCACCGGGGTGGTGCCCATCCTGGTCGAGCTGGAC GGCGACGTAAACGGCCACAAGTTCAGCGTGTCCGGCGAGGG CGAGGGCGATGCCACCTACGGCAAGCTGACCCTGAAGTTCA TCTGCACCACCGGCAAGCTGCCCGTGCCCTGGCCCACCCTC GTGACCACCCTGACCTACGGCGTGCAGTGCTTCAGCCGCTA CCCCGACCACATGAAGCAGCACGACTTCTTCAAGTCCGCCA | 3 |
| | TGCCCGAAGGCTACGTCCAGGAGCGCACCATCTTCTTCAAG GACGACGGCAACTACAAGACCCGCGCCGAGGTGAAGTTCGA GGGCGACACCCTGGTGAACCGCATCGAGCTGAAGGGCATCG ACTTCAAGGAGGACGGCAACATCCTGGGGCACAAGCTGGAG TACAACTACAACAGCCACAACGTCTATATCATGGCCGACAA GCAGAAGAACGGCATCAAGGTGAACTTCAAGATCCGCCACA ACATCGAGGACGGCAGCGTGCAGCTCGCCGACCACTACCAG CAGAACACCCCCATCGGCGACGGCCCCGTGCTGCTGCCCGA CAACCACTACCTGAGCACCCAGTCCGCCCTGAGCAAAGACC CCAACGAGAAGCGCGATCACATGGTCCTGCTGGAGTTCGTG ACCGCCGCCGGGATCACTCTCGGCATGGACGAGCTGTACAA GTAAAGCGGCCGCGACTCTAGATCATAATCAGCCATACCAC ATTTGTAGAGGTTTTACTTGCTTTAAAAAACCTCCCACACC TCCCCCTGAACCTGAAACATAAAATGAATGCAATTGTTGTT GTTAACTTGTTTATTGCAGCTTATAATGGTTACAAATAAAG CAATAGCATCACAAATTTCACAAATAAAGCATTTTTTTCAC TGCATTCTAGTTGTGGTTTGTCCAAACTCATCAATGTATCT TAAGGCGTAAATTGTAAGCGTTAATATTTTGTTAAAATTCG CGTTAAATTTTTGTTAAATCAGCTCATTTTTTAACCAATAG GCCGAAATCGGCAAAATCCCTTATAAATCAAAAGAATAGAC CGAGATAGGGTTGAGTGTTGTTCCAGTTTGGAACAAGAGTC CACTATTAAAGAACGTGGACTCCAACGTCAAAGGGCGAAAA ACCGTCTATCAGGGCGATGGCCCACTACGTGAACCATCACC CTAATCAAGTTTTTTGGGGTCGAGGTGCCGTAAAGCACTAA ATCGGAACCCTAAAGGGAGCCCCCGATTTAGAGCTTGACGG GGAAAGCCGGCGAACGTGGCGAGAAAGGAAGGGAAGAAAGC GAAAGGAGCGGGCGCTAGGGCGCTGGCAAGTGTAGCGGTCA CGCTGCGCGTAACCACCACACCCGCCGCGCTTAATGCGCCG CTACAGGGCGCGTCAGGTGGCACTTTTCGGGGAAATGTGCG CGGAACCCCTATTTGTTTATTTTTCTAAATACATTCAAATA TGTATCCGCTCATGAGACAATAACCCTGATAAATGCTTCAA TAATATTGAAAAAGGAAGAGTCCTGAGGCGGAAAGAACCAG CTGTGGAATGTGTGTCAGTTAGGGTGTGGAAAGTCCCCAGG CTCCCCAGCAGGCAGAAGTATGCAAAGCATGCATCTCAATT AGTCAGCAACCAGGTGTGGAAAGTCCCCAGGCTCCCCAGCA GGCAGAAGTATGCAAAGCATGCATCTCAATTAGTCAGCAAC CATAGTCCCGCCCCTAACTCCGCCCATCCCGCCCCTAACTC CGCCCAGTTCCGCCCATTCTCCGCCCCATGGCTGACTAATT TTTTTTATTTATGCAGAGGCCGAGGCCGCCTCGGCCTCTGA GCTATTCCAGAAGTAGTGAGGAGGCTTTTTTGGAGGCCTAG GCTTTTGCAAAGATCGATCAAGAGACAGGATGAGGATCGTT TCGCATGATTGAACAAGATGGATTGCACGCAGGTTCTCCGG CCGCTTGGGTGGAGAGGCTATTCGGCTATGACTGGGCACAA CAGACAATCGGCTGCTCTGATGCCGCCGTGTTCCGGCTGTC AGCGCAGGGGCGCCCGGTTCTTTTTGTCAAGACCGACCTGT CCGGTGCCCTGAATGAACTGCAAGACGAGGCAGCGCGGCTA TCGTGGCTGGCCACGACGGGCGTTCCTTGCGCAGCTGTGCT | |
| | CGACGTTGTCACTGAAGCGGGAAGGGACTGGCTGCTATTGG GCGAAGTGCCGGGGCAGGATCTCCTGTCATCTCACCTTGCT CCTGCCGAGAAAGTATCCATCATGGCTGATGCAATGCGGCG GCTGCATACGCTTGATCCGGCTACCTGCCCATTCGACCACC AAGCGAAACATCGCATCGAGCGAGCACGTACTCGGATGGAA GCCGGTCTTGTCGATCAGGATGATCTGGACGAAGAGCATCA GGGGCTCGCGCCAGCCGAACTGTTCGCCAGGCTCAAGGCGA GCATGCCCGACGGCGAGGATCTCGTCGTGACCCATGGCGAT GCCTGCTTGCCGAATATCATGGTGGAAAATGGCCGCTTTTC TGGATTCATCGACTGTGGCCGGCTGGGTGTGGCGGACCGCT ATCAGGACATAGCGTTGGCTACCCGTGATATTGCTGAAGAG CTTGGCGGCGAATGGGCTGACCGCTTCCTCGTGCTTTACGG TATCGCCGCTCCCGATTCGCAGCGCATCGCCTTCTATCGCC TTCTTGACGAGTTCTTCTGAGCGGGACTCTGGGGTTCGAAA TGACCGACCAAGCGACGCCCAACCTGCCATCACGAGATTTC GATTCCACCGCCGCCTTCTATGAAAGGTTGGGCTTCGGAAT CGTTTTCCGGGACGCCGGCTGGATGATCCTCCAGCGCGGGG ATCTCATGCTGGAGTTCTTCGCCCACCCTAGGGGGAGGCTA ACTGAAACACGGAAGGAGACAATACCGGAAGGAACCCGCGC TATGACGGCAATAAAAAGACAGAATAAAACGCACGGTGTTG GGTCGTTTGTTCATAAACGCGGGGTTCGGTCCCAGGGCTGG CACTCTGTCGATACCCCACCGAGACCCCATTGGGGCCAATA CGCCCGCGTTTCTTCCTTTTCCCCACCCCACCCCCCAAGTT CGGGTGAAGGCCCAGGGCTCGCAGCCAACGTCGGGGCGGCA GGCCCTGCCATAGCCTCAGGTTACTCATATATACTTTAGAT TGATTTAAAACTTCATTTTTAATTTAAAAGGATCTAGGTGA AGATCCTTTTTGATAATCTCATGACCAAAATCCCTTAACGT GAGTTTTCGTTCCACTGAGCGTCAGACCCCGTAGAAAAGAT CAAAGGATCTTCTTGAGATCCTTTTTTTCTGCGCGTAATCT GCTGCTTGCAAACAAAAAAACCACCGCTACCAGCGGTGGTT TGTTTGCCGGATCAAGAGCTACCAACTCTTTTTCCGAAGGT AACTGGCTTCAGCAGAGCGCAGATACCAAATACTGTCCTTC TAGTGTAGCCGTAGTTAGGCCACCACTTCAAGAACTCTGTA GCACCGCCTACATACCTCGCTCTGCTAATCCTGTTACCAGT GGCTGCTGCCAGTGGCGATAAGTCGTGTCTTACCGGGTTGG ACTCAAGACGATAGTTACCGGATAAGGCGCAGCGGTCGGGC TGAACGGGGGGTTCGTGCACACAGCCCAGCTTGGAGCGAAC GACCTACACCGAACTGAGATACCTACAGCGTGAGCTATGAG AAAGCGCCACGCTTCCCGAAGGGAGAAAGGCGGACAGGTAT CCGGTAAGCGGCAGGGTCGGAACAGGAGAGCGCACGAGGGA GCTTCCAGGGGGAAACGCCTGGTATCTTTATAGTCCTGTCG GGTTTCGCCACCTCTGACTTGAGCGTCGATTTTTGTGATGC TCGTCAGGGGGGCGGAGCCTATGGAAAAACGCCAGCAACGC GGCCTTTTTACGGTTCCTGGCCTTTTGCTGGCCTTTTGCTC ACATGTTCTTTCCTGCGTTATCCCCTGATTCTGTGGATAAC CGTATTACCGCCATGCAT | |

### <Example 2>

The modifying composition 1 produced in Example 1 was used to perform cleavage experiment of the genome DNA 4 (SEQ. ID. 9) coding the GFP in the following procedure. FIG. 5 is a diagram illustrating the overview of Example 2.

### (1) PCR amplification of GFP

A template having SEQ. ID. 3 was used, and the following primer was used to obtain a PCR product of the GFP indicated by SEQ. ID. 9 by an ordinary method.
- Fw primer (5'-GTGAGCAAGGGCGAGGAGCTG-3': SEQ. ID. 10)
- Rv primer (5'-CTTGTACAGCTCGTCCATGCCG-3': SEQ. ID. 11)

**[Table 5]**

| NAME | SEQUENCE | SEQ. ID. |
|---|---|---|
| GFP | GTGAGCAAGGGCGAGGAGCTGTTCACCGGGGTGGTGCCCAT CCTGGTCGAGCTGGACGGCGACGTAAACGGCCACAAGTTCA GCGTGTCCGGCGAGGGCGAGGGCGATGCCACCTACGGCAAG CTGACCCTGAAGTTCATCTGCACCACCGGCAAGCTGCCCGT GCCCTGGCCCACCCTCGTGACCACCCTGACCTACGGCGTGC AGTGCTTCAGCCGCTACCCCGACCACATGAAGCAGCACGAC TTCTTCAAGTCCGCCATGCCCGAAGGCTACGTCCAGGAGCG CACCATCTTCTTCAAGGACGACGGCAACTACAAGACCCGCG CCGAGGTGAAGTTCGAGGGCGACACCCTGGTGAACCGCATC GAGCTGAAGGGCATCGACTTCAAGGAGGACGGCAACATCCT GGGGCACAAGCTGGAGTACAACTACAACAGCCACAACGTCT ATATCATGGCCGACAAGCAGAAGAACGGCATCAAGGTGAAC TTCAAGATCCGCCACAACATCGAGGACGGCAGCGTGCAGCT CGCCGACCACTACCAGCAGAACACCCCCATCGGCGACGGCC CCGTGCTGCTGCCCGACAACCACTACCTGAGCACCCAGTCC GCCCTGAGCAAAGACCCCAACGAGAAGCGCGATCACATGGT CCTGCTGGAGTTCGTGACCGCCGCCGGGATCACTCTCGGCA TGGACGAGCTGTACAAG | 9 |

(2) 200 ng of the PCR product obtained in above (1), 200 ng of the RNA produced in Example 1, and 125 ng of the fusion protein (Nova-FokI) produced in Example 1 were used. CutSmart Buffer was used to perform treatment at 37 °C for 2 hours, then RNase treatment was performed, and electrophoresis was performed. FIG. 6 illustrates the result. Each lane of an electrophoresis photograph illustrated in FIG. 6 is as follows.
M: 100 bp Marker
1: only PCR product of GFP
2: PCR product of GFP + fusion protein
3: PCR product of GFP + fusion protein + RNA

As illustrated in FIG. 6, in "PCR product of GFP + fusion protein" of Lane 2, it was confirmed that the PCR product was cleaved at random by FokI. In contrast, in "PCR product of GFP + fusion protein + RNA" of Lane 3, it was confirmed that the PCR product was cleaved by a predetermined length, because the hybridization region 21 of the modifying composition 1 hybridizes to the PCR product of the GFP.

Note that, as illustrated in FIG. 5, the hybridization region 21 was designed so as to be cleaved near substantially the center of the sequence coding the GFP (714 bp). Thus, an experiment was performed to confirm that the DNA fragment indicated by the arrow to Lane 3 of FIG. 6 includes both the upstream and downstream of the sequence coding the GFP.

The band indicated by the arrow to Lane 3 of FIG. 6 was gel-extracted using QIAquick Gel Extraction Kit by QIAGEN K.K. (hereafter, referred to as "band extract"). The overview of the confirmation experiment will be described with reference to FIG. 5.
(1) Amplification of a band extract
   1: Fw primer having SEQ. ID. 10 and Rv primer having SEQ. ID. 12 were used to amplify the band extract. The length of the amplification product was 221 bp.
   2: Fw primer having SEQ. ID. 13 and Rv primer having SEQ. ID. 11 were used to amplify the band extract. The length of the amplification product was 251 bp.
(2) Amplification of a GFP fragment with a template having SEQ. ID. 3
   3: Fw primer having SEQ. ID. 10 and Rv primer having SEQ. ID. 12 were used to amplify the sequence coding the GFP fragment. The length of the amplification product was 221 bp.
   4: Fw primer having SEQ. ID. 13 and Rv primer having SEQ. ID. 11 were used to amplify the sequence coding the GFP fragment. The length of the amplification product was 251 bp.

Note that SEQ. IDs. 12 and 13 are as follows.
- SEQ. ID. 12: 5'-TAGCGGCTGAAGCACTGCAC-3'
- SEQ. ID. 13: 5'-ACAAGCAGAAGAACGGCATCAAG-3'

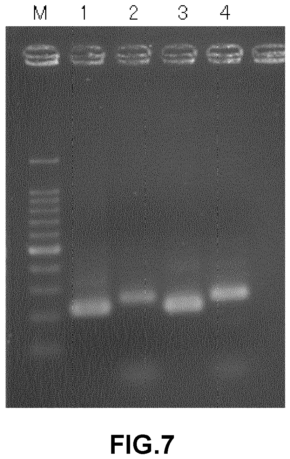

Electrophoresis of the above amplification products was performed. FIG. 7 illustrates the result. Lane M of FIG. 7 represents 100 bp marker, and Lanes 1 to 4 of FIG. 7 correspond to amplification products described in above 1 to 4. It was confirmed from the result indicated by Lanes 1 and 2 of FIG. 7 that the band extract cleaved by the fusion protein 3 includes the upstream and downstream of the sequence coding the GFP fragment. In accordance with the above result, it was confirmed that the use of the modifying composition disclosed in the present application enables modification of a nucleic acid sequence without depending on a PAM sequence or a PFS sequence.

### [Industrial Applicability]

With the use of the nucleic acid sequence modifying composition and the method for modifying a target site of a nucleic acid sequence disclosed in the present application, it is possible to modify a nucleic acid sequence without depending on a PAM sequence or a PFS sequence. Therefore, the nucleic acid sequence modifying composition and the method for modifying a target site of a nucleic acid sequence disclosed in the present application are useful for industries that require genome editing, such as pharmaceutical industry, research institutions, or the like.

### [List of Reference Numerals]

- 1: genome DNA modifying composition
- 2: RNA
- 21: hybridization region
- 22: guide region
- 23: recognition region
- 24: first complementary region
- 3: fusion protein
- 31: binding domain
- 32: modification domain
- 4: nucleic acid sequence, genome DNA, RNA
- 41: target site

### [List of Sequences]

M21062PCT.xml

## Claims

1. A nucleic acid sequence modifying composition comprising RNA and a fusion protein,
wherein the RNA includes
a hybridization region adapted to hybridize to a sequence on the 5' side or the 3' side of a target site of a nucleic acid sequence, and
a guide region that guides the fusion protein,
wherein the guide region includes at least one recognition region that forms a complex with the fusion protein, and
wherein the fusion protein includes
a binding domain that recognizes the recognition region of the RNA and forms a complex with the recognition region, where it is excluded that the binding domain includes an RNA recognition region of a Cas protein group, and
a modification domain that modifies the target site of the nucleic acid sequence.

2. The nucleic acid sequence modifying composition according to claim 1, wherein the fusion protein includes a linker sequence that links the binding domain and the modification domain to each other.

3. The nucleic acid sequence modifying composition according to claim 1 or 2, wherein the guide region includes two recognition regions.

4. The nucleic acid sequence modifying composition according to any one of claims 1 to 3 further comprising a first complementary region connected to one end of the hybridization region,
wherein the hybridization region and the guide region are indirectly connected to each other when the first complementary region forms a complementary pair with one end side of the guide region.

5. The nucleic acid sequence modifying composition according to any one of claims 1 to 4, wherein the guide region includes a stem loop.

6. The nucleic acid sequence modifying composition according to any one of claims 1 to 5,
wherein the binding domain includes an RNA binding region of Nova, and
wherein the modification domain includes a cleavage region of FokI that cleaves genome DNA.

7. The nucleic acid sequence modifying composition according to any one of claims 1 to 6, wherein the nucleic acid sequence is genome DNA.

8. A nucleic acid sequence modifying composition comprising:
a nucleic acid that serves as a template used for transcribing the RNA according to any one of claims 1 to 7; and
a nucleic acid that serves as a template used for translating the fusion protein according to any one of claims 1 to 7.

9. RNA for forming the nucleic acid sequence modifying composition according to any one of claims 1 to 7.

10. A nucleic acid that serves as the template used for transcribing the RNA for forming the nucleic acid sequence modifying composition according to claim 8.

11. A fusion protein for forming the nucleic acid sequence modifying composition according to any one of claims 1 to 7.

12. A nucleic acid that serves as the template used for translating the fusion protein for forming the nucleic acid sequence modifying composition according to claim 8.

13. A method for modifying a target site of a nucleic acid sequence, the method comprising:
an introduction step of introducing the nucleic acid sequence modifying composition according to any one of claims 1 to 8 into a cell; and
a modification step of the modification domain modifying the target site of the nucleic acid sequence.

14. The method according to claim 13 further comprising, before the introduction step, a hybridization region determination step of determining a hybridization region adapted to hybridize to a sequence on the 5' side or the 3' side of the target site of the nucleic acid sequence.

15. The method according to claim 13 or 14, wherein one hybridization region is required for modifying one target site.

16. The method according to claim 15, wherein the guide region includes two recognition regions.

17. The method according to claim 16 further comprising, before the introduction step, a guide region designing step of designing at least the number and an RNA sequence of recognition regions so as to be able to form a complex with a required number of fusion proteins for modification of the nucleic acid sequence.
